# FASCICULE DE BREVET EUROPEEN

(11) **EP 0 748 623 B1**
(45) Date de publication et mention de la délivrance du brevet: **21.05.1997**
(21) Numéro de dépôt: 96401102.7
(22) Date de dépôt: 21.05.1996
(51) Int. Cl.: A61K 7/42

(54) **Compositions cosmétiques photoprotectrices contenant des composés amides**
Kosmetische Lichtschutzmittel, die Amidverbindungen enthalten
Cosmetic sunscreen compositions containing amide compounds

(30) Priorité: 16.06.1995 FR 9507245
(43) Date de publication de la demande: 18.12.1996
(73) Titulaire: L'OREAL, 75008 Paris (FR)
(72) Inventeur: Ascione, Jean-Marc, 75018 Paris (FR); Sterle, Pascal, 95230 Soisy S/ Montmorency (FR)
(74) Mandataire: Andral, Christophe André Louis

(56) Documents cités:
- EP-A- 0 457 687
- EP-A- 0 517 104

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires à pouvoir photoprotecteur amélioré, comprenant, dans un support cosmétiquement acceptable en particulier de type émulsion huile-dans-eau, (i) de la 2,4,6-tris[p-(2'-éthylhexyl-1'-oxycarbonyl)anilino]-1,3,5-triazine à titre de filtre organique solaire lipophile et (ii) une ou plusieurs huiles particulières choisies au sein des composés amidés.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV). Selon leur caractère lipophile ou au contraire hydrophile, ces filtres peuvent se répartir, respectivement, soit dans la phase grasse, soit dans la phase aqueuse, de la composition finale.

Il s'avère qu'un filtre particulièrement intéressant et largement utilisé à ce jour est constitué par la 2,4,6-tris[p-(2'-éthylhexyl-1'-oxycarbonyl)anilino]-1,3,5-triazine (voir EP-A-0 457 687 et EP-A-0 517 104), qui est un filtre lipophile, fortement actif dans l'UV-B, photostable et rémanent à l'eau, et qui est notamment vendu sous la dénomination commerciale de "UVINUL T 150" par la Société BASF. Malgré tout, en l'absence de renfort d'autres filtres, son pouvoir photoprotecteur est assez limité dans les supports cosmétiques habituels contenant des huiles telles que les mono- ou polyalcools gras oxyéthylénés ou oxypropylénés ("CETIOL HE" de chez Henkel, ou "WITCONOL APM" de chez Witco).

La présente invention vise à résoudre le problème ci-dessus.

Ainsi, à la suite d'importantes recherches menées dans le domaine de la photoprotection évoqué ci-dessus, la Demanderesse a maintenant découvert, de façon inattendue et surprenante, qu'il était possible d'améliorer de manière substantielle le pouvoir photoprotecteur de la 2,4,6-tris[p-(2'-éthylhexyl-1'-oxycarbonyl)anilino]-1,3,5-triazine contenue dans les compositions cosmétiques antisolaires en associant ce filtre particulier avec au moins une huile choisie au sein des composés amidés.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) de la 2,4,6-tris[p-(2'-éthylhexyl-1'-oxycarbonyl)anilino]-1,3,5-triazine, à titre de filtre, et (ii) au moins une huile choisie parmi les composés amidés.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

Un autre objet enfin de la présente invention réside dans l'utilisation d'une huile telle que définie ci-dessus pour améliorer le pouvoir photoprotecteur de la 2,4,6-tris[p-(2'-éthylhexyl-1'-oxycarbonyl)anilino]-1,3,5-triazine contenue dans une composition cosmétique antisolaire.

D'autres caractéristiques, aspects et avantages de la présente invention apparaîtront à la lecture de la description détaillée qui va suivre.

Comme indiqué précédemment, la 2,4,6-tris[p-(2'-éthylhexyl-1'-oxycarbonyl)anilino]-1,3,5-triazine est un filtre connu en soi, actif dans l'UV-B, se présentant sous une forme solide, et qui est vendu notamment sous la dénomination commerciale de "UVINUL T 150" par la Société BASF. Ce produit répond à la formule (5) suivante : 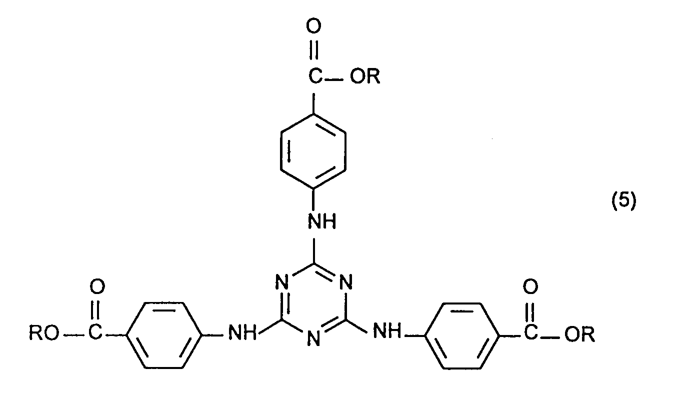 dans laquelle R désigne un radical 2-éthyl hexyle.

Ce filtre peut être présent dans les compositions selon l'invention à une concentration comprise entre 0,1 et 10% en poids, et de préférence entre 0,2 et 5% en poids, par rapport au poids total de la composition.

Au sens de la présente invention, on entend par composé amidé tout composé présentant dans sa structure chimique au moins un groupement (ou fonction) amide

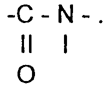

Les composés amidés plus particulièrement visés par la présente invention sont ceux répondant à la formule (1) suivante : 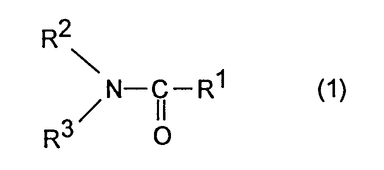 dans laquelle les radicaux R¹, R² et R³, qui peuvent être identiques ou différents, représentent l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses, étant entendu que, dans cette formule, le radical R¹ peut former avec le radical R² ou avec le radical R³ un cycle contenant inclusivement de 5 à 18 atomes de carbone, et que les radicaux R² et R³ peuvent ensemble former un cycle contenant de 5 à 18 atomes de carbone, bornes incluses.

Comme exemples de radicaux hydrocarbonés saturés aliphatiques, on peut notamment citer les radicaux alkyle, linéaires ou ramifiés, substitués ou non, en C₁-C₃₀, de préférence en C₁-C₂₂, et en particulier les radicaux méthyle, éthyle, n-propyle, isopropyle, n-butyle, isobutyle, ter.-butyle, pentyle, n-amyle, isoamyle, néopentyle, n-hexyle, n-heptyle, n-octyle, 2-éthylhexyle, ter.-octyle, décyle, lauryle et octadécyle.

A titre d'exemples de radicaux hydrocarbonés saturés cycliques, on peut notamment citer les radicaux cyclopentyle et cyclohexyle, éventuellement substitués, en particulier par des radicaux alkyle.

Comme exemples de radicaux hydrocarbonés insaturés aliphatiques, on peut notamment citer les radicaux alcényle ou alcynyle, linéaires ou ramifiés, substitués ou non, en C₂-C₃₀, de préférence en C₂-C₂₂, et particulier les radicaux vinyle, allyle, oléyle et linoléyle.

Comme exemples de radicaux hydrocarbonés insaturés cycliques, on peut notamment citer les radicaux aryle tels que phényle et naphtyle, éventuellement substitués, en particulier par des alkyle, comme par exemple le radical tolyle, et à titre d'exemples de radicaux cycloaliphatiques insaturés, on peut citer plus particulièrement les radicaux benzyle et phényléthyle.

Par radicaux fonctionnalisés, on entend plus particulièrement des radicaux comportant dans leur structure chimique, tant dans la chaîne principale que sur un chaînon secondaire, un ou plusieurs groupements fonctionnels du type notamment esters, éthers, alcools, amines, amides et cétones, mais de préférence esters.

Parmi les composés amidés de formule (1) convenant bien à la présente invention, on préfère plus particulièrement mettre en oeuvre les composés présentant au moins l'une, et encore plus préférentiellement l'ensemble, des caractéristiques suivantes :
- le composé amidé est un amide N-substitué, et encore plus préférentiellement N,N-disubstitué,
- R¹ est un radical alkyle, linéaire ou ramifié, de préférence en C₁-C₂₂ et encore plus préférentiellement en C₁-C₁₂, ou bien encore un radical phényle lui-même éventuellement substitué par un ou plusieurs radicaux alkyles, linéaires ou ramifiés, en C₁-C₁₂,
- R² est un radical alkyle, linéaire ou ramifié, de préférence en C₁-C₂₂ et encore plus préférentiellement en C₁-C₁₂,
- R³ est un radical alkyle, linéaire ou ramifié, choisi au sein de ceux définis pour R², ou bien encore représente un radical monovalent à fonction ester répondant à la formule (2) suivante : 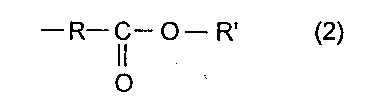 dans laquelle R et R', qui peuvent être identiques ou différents, représentent deux radicaux hydrocarbonés, de préférence de type alkyle, contenant de 1 à 12 atomes de carbone, de préférence de 1 à 8 atomes de carbone.

Selon un mode particulier de réalisation de la présente invention, les composés amidés précédemment définis que l'on met en oeuvre sont des corps gras liquides à température ordinaire. De préférence, ce sont des huiles qui présentent par ailleurs une bonne solubilité dans les phases grasses habituellement utilisées pour la préparation de supports cosmétiquement acceptables.

A titre d'exemples d'huiles amidées spécifiques qui se sont avérées présenter des propriétés absolument remarquables dans l'amélioration du pouvoir photoprotecteur de la 2,4,6-tris[p-(2'-éthylhexyl-1'-oxycarbonyl)anilino]-1,3,5-triazine, on peut plus particulièrement citer :
- les N,N-diéthyl-méthylbenzamides de formule (3) suivante : 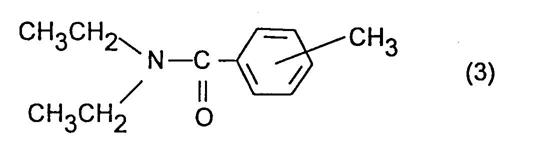 dont le N,N-dièthyl-3-méthylbenzamide,
- le N-butyl, N-acétyl aminopropionate d'éthyle de formule (4) suivante :

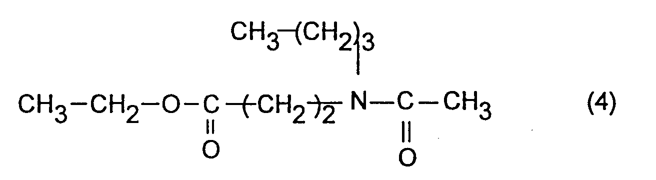

Les huiles amidées utilisées conformément à la présente invention sont généralement présentes dans les compositions antisolaires finales à des teneurs comprises entre 0,5 et 50% en poids par rapport au poids total de ladite composition, et de préférence à des teneurs comprises entre 1 et 30% en poids.

D'une manière générale, on notera que les concentrations en filtre et en huile(s) sont choisies de manière telle que le facteur de protection solaire de la composition finale soit de préférence d'au moins 2.

Enfin, toujours selon un mode préféré de réalisation de la présente invention, le support cosmétiquement acceptable dans lequel se trouvent contenus les filtres et les huiles conformes à l'invention est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que le filtre mentionné ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants et notamment les antioxydants ARL, les opacifiants, les stabilisants, les émollients, les silicones , les α-hydroxyacides, les agents anti-mousse, les agents hydratants, les vitamines, les parfums, les conservateurs, les tensio-actifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir ce ou ces éventuels composés complémentaires (filtres, pigments, adjuvants, ...) et/ou leurs quantités de manière telle que les propriétés avantageuses attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel-crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant les filtres ou autres adjuvants hydrophiles) représente généralement de 50 à 95% en poids, de préférence de 70 à 90% en poids, par rapport à l'ensemble de la formulation, la phase huileuse (comprenant notamment le ou les filtres et autres adjuvants lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation. Si on le désire, la phase grasse de l'émulsion peut n'être constituée que par des huiles conformes à l'invention telles que précédemment définies.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE 1:

On a préparé diverses formulations antisolaires se présentant sous la forme d'émulsions H/E et contenant la 2,4,6-tris[p-(2'-éthylhexyl-1'-oxycarbonyl)anilino]-1,3,5-triazine en présence de différentes huiles, à savoir des huiles amidées conformes à l'invention pour les compositions 1 et 2, et une huile classique correspondant à l'état de la technique pour la composition 3.

Dans les formulations suivantes, les quantités sont exprimées en % de poids par rapport au poids total de la composition :

| | |
|---|---|
| a) 2,4,6-tris[p-(2'-éthylhexyl-1'-oxycarbonyl)anilino]-1,3,5-triazine vendue sous la dénomination 〈〈 Uvinul T 150 〉〉 par BASF (filtre) | 8% |
| b) huile | 15% |
| c) terpolymère réticulé : acide méthacrylique/acrylate d'éthyle/steareth-10 allyl ether, en émulsion aqueuse à 30 % vendu sous la dénomination 〈〈 Salcare SC 90 〉〉 par Allied Colloids (émulsionnant) | 1,5% |
| d) triéthanolamine | 0,75% |
| e) conservateurs | qs |
| f) eau distillée | qsp 100% |

Pour la formulation 1, l'huile utilisée était le N-butyl, N-acétyl aminopropionate d'éthyle vendu sous la dénomination 〈〈 R 3535 〉〉 par Merck.

Pour la formulation 2, l'huile utilisée était le N,N diéthyl-3-méthylbenzamide vendu sous la dénomination 〈〈 DEET 〉〉 par Unipex.

Pour la formulation comparative 3, l'huile utilisée était le PEG-7 Glyceryl Cocoate vendu sous la dénomination 〈〈 Cétiol HE 〉〉 par Henkel.

Chacune de ces émulsions a été réalisée en dissolvant le filtre dans la phase grasse, puis en ajoutant l'émulsionnant dans cette phase grasse portée aux environs de 80°C, et enfin en additionnant sous agitation rapide la phase aqueuse préalablement chauffée à cette même température.

Pour chacune des formulations ainsi préparées, on a ensuite déterminé le facteur de protection solaire (SPF) qui lui était attaché. Le SPF a été déterminé in vitro suivant la méthode décrite par B.L. DIFFEY et al dans J. Soc. Cosmet. Chem. 40-127-133 (1989) ; cette méthode consiste à déterminer les facteurs de protection monochromatiques tous les 5 nm dans une gamme de longueurs d'onde de 290 nm à 400 nm et à calculer à partir de ceux-ci le facteur de protection solaire selon une équation mathématique donnée.

Les résultats en facteur de protection solaire (SPF) moyen selon les différentes compositions sont consignés dans le tableau (I) ci-dessous, σ représentant l'écart-type :

**Tableau (I)**

| | Huile | SPF in vitro (σ) |
|---|---|---|
| Formule 1 (invention) | 〈〈 R3535 〉〉 | 13,0 (1,7) |
| Formule 2 (invention) | 〈〈 DEET 〉〉 | 8,8 (0,5) |
| Formule 3 (comparatif) | 〈〈 Cétiol HE 〉〉 | 3,1 (0,4) |

Ces résultats démontrent clairement la supériorité en terme de facteur de protection solaire des formules contenant les huiles conformes à l'invention.

### EXEMPLE 2:

On donne ci-dessous un exemple concret de composition antisolaire conforme à l'invention se présentant sous la forme d'une émulsion NIE : les quantités sont exprimées en % de poids par rapport au poids total de la composition.

| | |
|---|---|
| a) 2,4,6-tris[p-(2'-éthylhexyl-1'-oxycarbonyl)anilino]-1,3,5-triazine vendue sous la dénomination 〈〈 Uvinul T 150 〉〉 par BASF (filtre) | 3% |
| b) N-butyl, N-acétyl aminopropionate d'éthyle vendu sous la dénomination 〈〈 R 3535 〉〉 par MERCK | 7,5% |
| c) mélange monostéarate de glycérol / stéarate de polyéthyléneglycol (100OE) 50/50 vendu sous la dénomination 〈〈 Simulsol 165 〉〉 par SEPPIC | 1,5% |
| d) malate de di-(2-ethyl hexyle) vendu sous la dénomination 〈〈 Ceraphyl 45 〉〉 par ISP | 2,5% |
| e) oxyde de titane rutile traité par stéarate d'aluminium / alumine vendu sous la dénomination 〈〈 Titanium dioxyde MT 100T 〉〉 par TAYCA | 2% |
| f) copolymère acide acrylique / acrylate d'éthyle réticulé en dispersion aqueuse à 28% vendu sous la dénomination 〈〈 Acrysol 33 〉〉 par ROHM & HAAS (émulsionnant) | 7,1% |
| g) triéthanolamine | qs pH=7,0 |
| h) acide benzène 1,4-di(3-méthylidène-10 camphosulfonique) (filtre) | 0,5% |
| i) conservateurs, parfums | qs |
| j) eau | qs 100% |

## Revendications

1. Compositions cosmétiques à usage topique, en particulier pour la photoprotection de la peau et/ou des cheveux, caractérisées par le fait qu'elles comprennent, dans un support cosmétiquement acceptable, (i) de la 2,4,6-tris[p-(2'-éthylhexyl-1'-oxycarbonyl)anilino]-1,3,5-triazine, à titre de filtre, et (ii) au moins une huile choisie parmi les composés amidés.

2. Compositions selon la revendication 1, caractérisées par le fait que ladite huile est choisie parmi les composés amidés de formule (1) suivante : 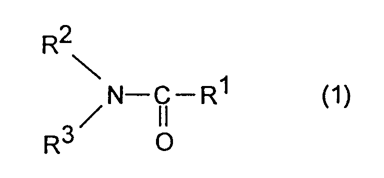 dans laquelle les radicaux R¹, R² et R³, qui peuvent être identiques ou différents, représentent l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses, étant entendu que, dans cette formule, le radical R¹ peut former avec le radical R² ou avec le radical R³ un cycle contenant inclusivement de 5 à 18 atomes de carbone, et que les radicaux R² et R³ peuvent ensemble former un cycle contenant de 5 à 18 atomes de carbone, bornes incluses.

3. Compositions selon la revendication 2, caractérisées par le fait que ladite huile est choisie parmi les composés amidés présentant au moins l'une des caractéristiques suivantes :
- le composé amidé est un amide N-substitué, et encore plus préférentiellement N,N-disubstitué,
- R¹ est un radical alkyle, linéaire ou ramifié, de préférence en C₁-C₂₂ et encore plus préférentiellement en C₁-C₁₂, ou bien encore un radical phényle luimême éventuellement substitué par un ou plusieurs radicaux alkyles, linéaires ou ramifiés, en C₁-C₁₂,
- R² est un radical alkyle, linéaire ou ramifié, de préférence en C₁-C₂₂ et encore plus préférentiellement en C₁-C₁₂,
- R³ est un radical alkyle, linéaire ou ramifié, choisi au sein de ceux définis pour R², ou bien encore représente un radical monovalent à fonction ester répondant à la formule (2) suivante : 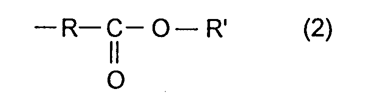 dans laquelle R et R', qui peuvent être identiques ou différents, représentent deux radicaux hydrocarbonés, de préférence de type alkyle, contenant de 1 à 12 atomes de carbone, de préférence de 1 à 8 atomes de carbone.

4. Compositions selon la revendication 3, caractérisées par le fait que ladite huile est choisie parmi les composés amidés présentant l'ensemble desdites caractéristiques.

5. Compositions selon la revendication 4, caractérisées par le fait que ladite huile est le N,N-diéthyl-3-méthylbenzamide.

6. Compositions selon la revendication 4, caractérisées par le fait que ladite huile est le N-butyl, N-acétyl aminopropionate d'éthyle.

7. Compositions selon l'une quelconque des revendications précédentes, caractérisées par le fait que ledit filtre est présent à une teneur comprise entre 0,1 et 10 % en poids par rapport au poids total de la composition.

8. Compositions selon la revendication 7, caractérisées par le fait que ladite teneur est comprise entre 0,2 et 5 % en poids.

9. Compositions selon l'une quelconque des revendications précédentes, caractérisées par le fait que la ou les huiles sont présentes à une teneur comprise entre 0,5 et 50 % en poids par rapport au poids total de la composition.

10. Compositions selon la revendication 9, caractérisées par le fait que ladite teneur est comprise entre 1 et 30 % en poids.

11. Compositions selon l'une quelconque des revendications précédentes, caractérisées par le fait que ledit support cosmétiquement acceptable se présente sous la forme d'une émulsion de type huile-dans-eau.

12. Compositions selon l'une quelconque des revendications précédentes, caractérisées par le fait qu'elles comprennent en outre un ou plusieurs filtres organiques complémentaires actifs dans l'UV-A et/ou UV-B, hydrophiles ou lipophiles.

13. Compositions selon la revendication 12, caractérisées par le fait que lesdits filtres organiques complémentaires sont choisis parmi les dérivés cinnamiques, les dérivés salicyliques, les dérivés du camphre, les dérivés de triazine, les dérivés de la benzophénone, les dérivés du dibenzoylméthane, les dérivés de β,β -diphénylacrylate, les dérivés de l'acide p-aminobenzoïque, les polymères filtres et silicones filtres.

14. Compositions selon l'une quelconque des revendications précédentes, caractérisées par le fait qu'elles comprennent en outre, à titre d'agents photoprotecteurs complémentaires, des pigments ou des nanopigments d'oxydes métalliques, enrobés ou non, capables de bloquer physiquement, par diffusion et/ou réflection, le rayonnement UV.

15. Compositions selon la revendication 14, caractérisées par le fait que lesdits pigments ou nanopigments sont choisis parmi les oxydes de titane, de zinc, de fer, de zirconium, de cérium et leurs mélanges, enrobés ou non.

16. Compositions selon l'une quelconque des revendications précédentes, caractérisées par le fait qu'elles comprennent en outre au moins un agent de bronzage et/ou de brunissage artificiel de la peau.

17. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants et notamment les antioxydants ARL, les opacifiants, les stabilisants, les émollients, les silicones , les α-hydroxyacides, les agents anti-mousse, les agents hydratants, les vitamines, les parfums, les conservateurs, les tensio-actifs, les charges, les séquestrants, les polymères, les propulseurs, les agents alcalinisants ou acidifiants, les colorants.

18. Compositions selon l'une quelconque des revendications précédentes, caractérisées par le fait qu'il s'agit de compositions protectrices de l'épiderme humain ou de compositions antisolaires et qu'elles se présentent sous forme de dispersions vésiculaires non ioniques, émulsions, en particulier émulsions de type huile-dans-eau, crèmes, laits, gels, gels-crèmes, suspensions, dispersions, bâtonnets solides, mousses ou sprays.

19. Compositions selon l'une quelconque des revendications 1 à 18, caractérisées par le fait qu'il s'agit de compositions de maquillage des cils, des sourcils ou de la peau et qu'elles se présentent sous forme solide ou pâteuse, anhydre ou aqueuse, d'émulsions, de suspensions ou de dispersions.

20. Compositions selon l'une quelconque des revendications 1 à 18, caractérisées par le fait qu'il s'agit de compositions destinées à la protection des cheveux contre les rayons ultraviolets et qu'elles se présentent sous forme de shampooings, de lotions, de gels, d'émulsions, de dispersions vésiculaires non ioniques ou de laques pour cheveux.

21. Compositions selon l'une quelconque des revendications précédentes, caractérisées par le fait qu'elles présentent un facteur de protection solaire sur peau d'au moins 2.

22. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

23. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 21.

24. Utilisation d'une huile amidée telle que définie à l'une des revendications 1 à 6 pour améliorer le pouvoir photoprotecteur d'une composition cosmétique antisolaire contenant de la 2,4,6-tris[p-(2'-éthylhexyl-1'-oxycarbonyl)anilino]-1,3,5-triazine.

## Claims

1. Cosmetic compositions for topical use, in particular for photoprotection of the skin and/or the hair, characterized in that they comprise, in a cosmetically acceptable support, (i) 2,4,6-tris[p-(2'-ethylhexyl-1'-oxycarbonyl)anilino]-1,3,5-triazine as screening agent and (ii) at least one oil chosen from amide-containing compounds.

2. Compositions according to Claim 1, characterized in that the said oil is chosen from the amide-containing compounds of formula (1) below: 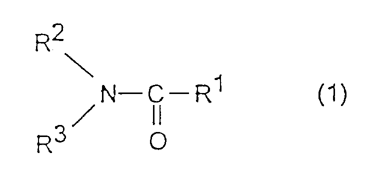 in which the radicals R¹, R² and R³, which may be identical or different, represent hydrogen or saturated or unsaturated, aliphatic, cycloaliphatic or cyclic monovalent hydrocarbon radicals, optionally functionalized, containing from 1 to 30 carbon atoms, preferably from 1 to 22 carbon atoms, limits included, it being understood that, in this formula, the radical R¹ may form with the radical R² or with the radical R³ a ring containing from 5 to 18 carbon atoms inclusive, and that the radicals R² and R³ may together form a ring containing from 5 to 18 carbon atoms, limits included.

3. Compositions according to Claim 2, characterized in that the said oil is chosen from amide-containing compounds having at least one of the following characteristics:
- the amide-containing compound is an N-substituted amide, and even more preferably an N,N-disubstituted amide,
- R¹ is a linear or branched, preferably C₁-C₂₂, and even more preferably C₁-C₁₂, alkyl radical or alternatively a phenyl radical which is itself optionally substituted with one or more linear or branched C₁-C₁₂ alkyl radicals,
- R² is a linear or branched, preferably C₁-C₂₂, and even more preferably C₁-C₁₂, alkyl radical,
- R³ is a linear or branched alkyl radical chosen from those defined for R², or alternatively represents a monovalent radical containing an ester function, corresponding to formula (2) below: 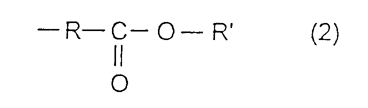 in which R and R', which may be identical or different, represent two hydrocarbon radicals, preferably of alkyl type, containing from 1 to 12 carbon atoms, preferably from 1 to 8 carbon atoms.

4. Compositions according to Claim 3, characterized in that the said oil is chosen from amide-containing compounds having all of the said characteristics.

5. Compositions according to Claim 4, characterized in that the said oil is N,N-diethyl-3-methylbenzamide.

6. Compositions according to Claim 4, characterized in that the said oil is ethyl N-butyl-N-acetylaminopropionate.

7. Compositions according to any one of the preceding claims, characterized in that the said screening agent is present at a content of between 0.1 and 10 % by weight relative to the total weight of the composition.

8. Compositions according to Claim 7, characterized in that the said content is between 0.2 and 5 % by weight.

9. Compositions according to any one of the preceding claims, characterized in that the oil or oils are present at a content of between 0.5 and 50 % by weight relative to the total weight of the composition.

10. Compositions according to Claim 9, characterized in that the said content is between 1 and 30 % by weight.

11. Compositions according to any one of the preceding claims, characterized in that the said cosmetically acceptable support is in the form of an emulsion of oil-in-water type.

12. Compositions according to any one of the preceding claims, characterized in that they also comprise one or more complementary hydrophilic or lipophilic organic screening agents which are active in the UV-A and/or UV-B range.

13. Compositions according to Claim 12, characterized in that the said complementary organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives, screening polymers and screening silicones.

14. Compositions according to any one of the preceding claims, characterized in that they also comprise, as complementary photoprotective agents, coated or uncoated metal oxide pigments or nanopigments capable of physically blocking UV radiation by diffusion and/or reflection.

15. Compositions according to Claim 14, characterized in that the said pigments or nanopigments are chosen from titanium oxide, zinc oxide, iron oxide, zirconium oxide, cerium oxide and mixtures thereof, which are coated or uncoated.

16. Compositions according to any one of the preceding claims, characterized in that they also comprise at least one agent for the artificial tanning and/or browning of the skin.

17. Compositions according to any one of the preceding claims, characterized in that they also comprise at least one adjuvant chosen from fatty substances, organic solvents, ionic or nonionic thickeners, softeners, antioxidants and in particular anti-free-radical antioxidants, opacifiers, stabilizers, emollients, silicones, α-hydroxy acids, anti-foaming agents, moisturizing agents, vitamins, fragrances, preserving agents, surfactants, fillers, sequestering agents, polymers, propellants, acidifying or basifying agents and dyes.

18. Compositions according to any one of the preceding claims, characterized in that they are compositions for protecting the human epidermis or antisun compositions and in that they are in the form of nonionic vesicle dispersions, emulsions, in particular emulsions of oil-in-water type, creams, milks, gels, cream-gels, suspensions, dispersions, solid sticks, foams or sprays.

19. Compositions according to any one of Claims 1 to 18, characterized in that they are compositions for making up the eyelashes, the eyebrows or the skin and in that they are in anhydrous or aqueous, solid or pasty form, or are in the form of emulsions, suspensions or dispersions.

20. Compositions according to any one of Claims 1 to 18, characterized in that they are compositions intended for protecting the hair against ultraviolet rays and in that they are in the form of shampoos, lotions, gels, emulsions, nonionic vesicle dispersions or lacquers for the hair.

21. Compositions according to any one of the preceding claims, characterized in that they have a sun protection factor of at least 2 on the skin.

22. Use of the compositions defined in any one of the preceding claims as, or for the manufacture of, cosmetic compositions for protection of the skin and/or the hair against ultraviolet radiation, in particular solar radiation.

23. Cosmetic treatment process for protecting the skin and/or the hair against ultraviolet radiation, in particular solar radiation, characterized in that it consists in applying an effective amount of a composition as defined in any one of Claims 1 to 21 to the skin and/or the hair.

24. Use of an amide-containing oil as defined in any one of Claims 1 to 6 in order to improve the photoprotective power of an antisun cosmetic composition containing 2,4,6-tris[p-(2'-ethylhexyl-1'-oxycarbonyl)anilino]-1,3,5-triazine.

## Patentansprüche

1. Kosmetische Zusammensetzungen zur topischen Anwendung, insbesondere zum Lichtschutz der Haut und/oder der Haare,
dadurch gekennzeichnet, daß
sie in einem kosmetisch akzeptablen Träger
(i) 2,4,6-Tris[p-(2'-ethylhexyl-1'-oxycarbonyl-)-anilino]-1,3,5-triazin als Lichtschutzfilter und
(ii) mindestens ein unter den Amidverbindungen ausgewähltes Öl enthalten.

2. Zusammensetzungen nach Anspruch 1, dadurch gekennzeichnet, daß das Öl unter Amidverbindungen der nachstehenden Formel (1) ausgewählt ist, 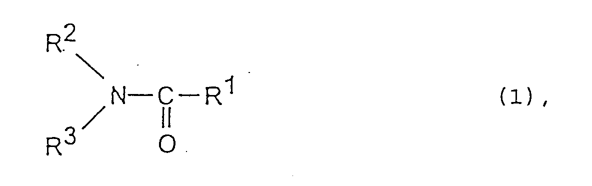 in der die Substituenten R¹, R² und R³, die gleich oder voneinander verschieden sein können, Wasserstoff oder einwertige gesättigte oder ungesättigte aliphatische, cycloaliphatische oder cyclische Kohlenwasserstoffgruppen mit 1 bis 30 Kohlenstoffatomen und vorzugsweise 1 bis 22 Kohlenstoffatomen, wobei die Grenzen eingeschlossen sind bedeuten, die gegebenenfalls funktionelle Gruppen aufweisen,
wobei in der obigen Formel der Substituent R¹ mit dem Substituenten R² oder mit dem Substituenten R³ mit 5 bis 8 Kohlenstoffatomen, wobei die Grenzen eingeschlossen sind, bilden kann und die Substituenten R² und R³ zusammen einen Ring mit 5 bis 18 Kohlenstoffatomen, wobei die Grenzen eingeschlossen sind, bilden kann.

3. Zusammensetzungen nach Anspruch 2, dadurch gekennzeichnet, daß das Öl unter Amidverbindungen ausgewählt ist, die mindestens eine der folgenden Eigenschaften aufweisen:
- die Amidverbindung ist ein N-substituiertes Amid und noch bevorzugter ein N,N-disubstituiertes Amid,
- R¹ ist eine geradkettige oder verzweigte Alkylgruppe mit vorzugsweise 1 bis 22 Kohlenstoffatomen und noch bevorzugter 1 bis 12 Kohlenstoffatomen oder eine Phenylgruppe, die ihrerseits gegebenenfalls mit einer oder mehreren geradkettigen oder verzweigten C₁₋₁₂ Alkylgruppen substituiert ist,
- R² ist eine geradkettige oder verzweigte Alkylgruppe mit vorzugsweise 1 bis 22 Kohlenstoffatomen und noch bevorzugter mit 1 bis 12 Kohlenstoffatomen,
- R³ ist eine geradkettige oder verzweigte Alkylgruppe, die unter den für R² definierten Gruppen ausgewählt ist, oder eine einwertige Gruppe mit einer Esterfunktion der nachstehenden Formel (2): 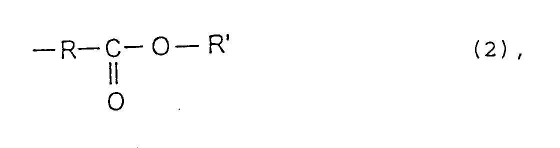 in der R und R', die gleich oder voneinander verschieden sein können, zwei Kohlenwasserstoffgruppen, vorzugsweise vom Alkyltyp, bedeuten, die 1 bis 12 Kohlenstoffatome und vorzugsweise 1 bis 8 Kohlenstoffatome aufweisen.

4. Zusammensetzung nach Anspruch 3, dadurch gekennzeichnet, daß das Öl unter den Amidverbindungen ausgewählt ist, welche sämtliche aufgeführten Eigenschaften aufweisen.

5. Zusammensetzungen nach Anspruch 4, dadurch gekennzeichnet, daß das Öl N,N-Diethyl-3-methylbenzamid ist.

6. Zusammensetzungen nach Anspruch 4, dadurch gekennzeichnet, daß das Öl N-Butyl,N-acetylaminopropionsäureethylester ist.

7. Zusammensetzungen nach einem der vorhergehenden Ansprüche, dadurch gekennzeichnet, daß der Gehalt an Lichtschutzfilter 0,1 bis 10 Gew.-%, bezogen auf das Gesamtgewicht der Zusammensetzung, beträgt.

8. Zusammensetzungen nach Anspruch 7, dadurch gekennzeichnet, daß der Gehalt an Lichtschutzfilter 0,2 bis 5 Gew.-% beträgt.

9. Zusammensetzungen nach einem der vorhergehenden Ansprüche, dadurch gekennzeichnet, daß das Öl bzw. die Öle in einem Mengenanteil von 0,5 bis 50 Gew.-%, bezogen auf das Gesamtgewicht der Zusammensetzung, vorliegen.

10. Zusammensetzungen nach Anspruch 9, dadurch gekennzeichnet, daß der Mengenanteil an Öl 1 bis 30 Gew.-% beträgt.

11. Zusammensetzungen nach einem der vorhergehenden Ansprüche, dadurch gekennzeichnet, daß der kosmetisch akzeptable Träger in Form ein Öl-in-Wasser-Emulsion vorliegt.

12. Zusammensetzungen nach einem der vorhergehenden Ansprüche, dadurch gekennzeichnet, daß sie ferner ein oder mehrere hydrophile oder lipophile zusätzliche organische Lichtschutzfilter enthalten, die im UV-A-Bereich oder im UV-B-Bereich wirksam sind.

13. Zusammensetzungen nach Anspruch 12, dadurch gekennzeichnet, daß die zusätzlichen organischen Lichtschutzfilter ausgewählt sind unter Zimtsäurederivaten, Salicylsäurederivaten, Campherderivaten, Triazinderivaten, Benzophenonderivaten, Dibenzoylmethanderivaten, β,β-Diphenylacrylat-Derivaten, p-Aminobenzosäurederivaten, polymeren Lichtschutzfiltern und Silicon-Lichtschutzfiltern.

14. Zusammensetzungen nach einem der vorhergehenden Ansprüche, dadurch gekennzeichnet, daß sie ferner als zusätzliche Lichtschutzstoffe Pigmente oder Nanopigmente von Metalloxiden enthalten, die gegebenenfalls ummantelt sind und befähigt sind, die UV-Strahlung physikalisch durch Streuung und/oder Reflexion zu blockieren.

15. Zusammensetzungen nach Anspruch 14, dadurch gekennzeichnet, daß die Pigmente oder Nanopigmente unter Titanoxid, Zinkoxid, Eisenoxid, Zirkoniumoxid und Ceroxid sowie deren Gemischen ausgewählt sind, wobei sie gegebenenfalls ummantelt sind.

16. Zusammensetzungen nach einem der vorhergehenden Ansprüche, dadurch gekennzeichnet, daß sie ferner mindestens ein künstliches Hautbräunungsmittel und/oder Hautbraunfärbungsmittel enthalten.

17. Zusammensetzungen nach einem der vorhergehenden Ansprüche, dadurch gekennzeichnet, daß sie ferner mindestens einen Hilfsstoff enthalten, der ausgewählt ist unter Fettstoffen, organischen Lösungsmitteln, ionischen oder nichtionischen Verdickungsmitteln, Mitteln für die Geschmeidigkeit, Antioxidantien und insbesondere ARL-Antioxidantien, Trübungsmitteln, Stabilisatoren, Mitteln für die Weichheit, Siliconen, α-Hydroxysäuren, Antischaummitteln, Hydratisierungsmitteln, Vitaminen, Parfums, Konservierungsmitteln, grenzflächenaktiven Mitteln, Füllstoffen, Sequestrierungsmitteln, Polymeren, Treibmitteln, Mitteln zum Alkalischmachen oder zum Ansäuern und Färbemitteln.

18. Zusammensetzungen nach einem der vorhergehenden Ansprüche, dadurch gekennzeichnet, daß es sich um Zusammensetzungen zum Schutz der menschlichen Epidermis oder um Zusammensetzungen zum Lichtschutz handelt und diese in Form von nichtionischen Vesikeldispersionen, Emulsionen, insbesondere Emulsionen vom Öl-in-Wasser-Typ, Cremen, Milchen, Gelen, Gelcremen, Suspensionen, Dispersionen, festen Sticks, Schäumen oder Sprays vorliegen.

19. Zusammensetzungen nach einem der Ansprüche 1 bis 18, dadurch gekennzeichnet, daß es sich um Zusammensetzungen zum Make-up der Wimpern, der Augenbrauen oder der Haut handelt und sie in fester oder pastoser, wasserfreier oder wasserhaltier Form, in Form von Emulsionen, Suspensionen oder Dispersionen vorliegen.

20. Zusammensetzungen nach einem der Ansprüche 1 bis 18, dadurch gekennzeichnet, daß es sich um Zusammensetzungen handelt, die zum Schutz der Haare gegen UV-Strahlung bestimmt sind und die in Form von Shampoos, Lotionen, Gelen, Emulsionen, nichtionischen Vesikeldispersionen oder Haarsprays vorliegen.

21. Zusammensetzungen nach einem der vorhergehenden Ansprüche, dadurch gekennzeichnet, daß sie einen Haut-Lichtschutzfaktor von mindestens 2 aufweisen.

22. Verwendung der in einem der vorhergehenden Ansprüche definierten Zusammensetzungen als oder zur Herstellung von kosmetischen Zusammensetzungen zum Schutz der Haut und/oder der Haare gegen UV-Strahlung, insbesondere gegen Sonnenstrahlung.

23. Verfahren zur kosmetischen Behandlung zum Schutz der Haut und/oder der Haare gegen UV-Strahlung, insbesondere gegen Sonnenstrahlung, dadurch gekennzeichnet, daß es darin besteht, auf die Haut und/oder die Haare eine wirksame Menge einer Zusammensetzung nach einem der Ansprüche 1 bis 21 aufzutragen.

24. Verwendung eines ölförmigen Amidverbindung nach einem der Ansprüche 1 bis 6 zur Verbesserung des Lichtschutzvermögens von kosmetischen Zusammensetzungen zum Lichtschutz, die 2,4,6-Tris[p-(2'ethylhexyl-1'oxycarbonyl-)-anilino]-1,3,5-triazin enthalten.
